Europäisches Patentamt

(19) European Patent Office

Office européen des brevets

(11) Publication number: **0 067 136**
**B1**

## (12) EUROPEAN PATENT SPECIFICATION

(45) Date of publication of patent specification: **28.08.85**

(51) Int. Cl.⁴: **A 61 K 9/20, A 61 K 31/665**

(21) Application number: **82830153.1**

(22) Date of filing: **02.06.82**

(54) **Pharmaceutical composition of phosphonomicin in tablet form.**

(30) Priority: **04.06.81 IT 2213381**

(43) Date of publication of application:
**15.12.82 Bulletin 82/50**

(45) Publication of the grant of the patent:
**28.08.85 Bulletin 85/35**

(84) Designated Contracting States:
**AT CH DE GB LI NL SE**

(56) References cited:
**GB-A-1 239 987**

**Remington's Pharmaceutical Sciences, pages
1576, 77 and 1580**

**The file contains technical information
submitted after the application was filed and
not included in this specification**

(73) Proprietor: **Crinos Industria Farmacobiologica
S.p.A.
Piazza XX Settembre, n. 2
I-22079 Villa Guardia Como (IT)**

(72) Inventor: **Ferro, Antonio
7, Via Maraini
Lugano (CH)**
Inventor: **Gazzani, Giovanni
28, Via Volta
Appiano Gentile (Como) (IT)**

(74) Representative: **Perani, Aurelio et al
c/o JACOBACCI-CASETTA & PERANI S.p.A 7,
Via Visconti di Modrone
I-20122 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a new pharmaceutical composition with antibiotic activity for oral use and, more particularly, to a composition in the form of tablets containing the antibiotic phosphonomycin as the active principle.

Phosphonomycin, or 1(−)-cis-(1,2-epoxypropyl) phosphonic acid, is a known antibiotic with a wide spectrum of activity, which may be administered both parenterally, in the form of its sodium salt, and orally, usually in the form of its calcium salt.

For oral administration, the calcium salt of phosphonomycin has been used until now in the form of capsules or tablets to be swallowed or in the form of a suspension for paediatric use (also to be swallowed). The capsules and tablets in general contain from 0.5 to 1.0 g of phosphonomycin (expressed as the acid) while the paediatric suspensions contain 0.05 g/ml of the active principle, again considered in the acid form. Daily doses for adults are usually 2 to 4 g and for children 0.9 to 2.25 g, divided into doses administered at regular intervals of 6 to 8 hours.

Although the pharmaceutical forms for oral administration known until now are more acceptable to patients than the forms for parenteral administration, they may not be used instead of the latter especially in more serious cases of infectious illnesses since it is known that the oral absorption of phosphonomycin is limited (about 60% is indeed eliminated unaltered in the faeces) and the levels obtainable in the blood are thus rather low and of rather short duration. This obviously limits the oral use of phosphonomycin to less serious forms of infection and also results in the need for frequent daily administrations in order to maintain minimum effective drug levels in the blood.

The applicant, supposing that phosphonomycin, by virtue of its small molecular dimensions, might have a good penetration index, even through the oral and oesophageal mucous membranes, which would be greater the longer the drug/tissue contact time, has found that by formulating phosphonomycin with pharmaceutically acceptable excipients in tablets to be sucked, arranged to dissolve slowly in the mouth, achieves a quicker and better absorption and higher drug levels in the blood than those achievable with the oral forms used up to now. This gives substantial advantages to the oral use of phosphonomycin in that it is possible, for example, to extend its use to more serious infections, while avoiding the inconveniences to the patients of parenteral administration and to achieve a local diffusion in the case of dental infections and infections in the oral cavity given the prolonged stay of the drug in the mouth, which advantageously enables the effects of its systemic action to be added to those of its local action. On the other hand, it is possible to produce equivalent results to those achieved with prior oral forms with the use of smaller doses and/or reduction in the number of daily administrations with the individual doses unchanged, while the use of the pharmaceutical form, comprising tablets to be sucked, allows the possibility of extending its use even to children, given the facility with which it is taken.

The tablets according to the present invention for sucking may be prepared with conventional formulations and by conventional methods, which produce tablets which can dissolve slowly in the oral cavity. According to the present invention, phosphonomycin is preferably used in the form of its calcium salt. Each tablet to be sucked may contain from 0.25 to 1.0 g of phosphonomycin (expressed as the acid) in addition to conventional excipients such as for example, saccharose, fructose, lactose; sweeteners such as ammonium glycyrrhizinate, saccharine and sugar itself; lubricants such as talc and magnesium stearate; dispersants such as lauryl sulphate, dioctyl sulphosuccinate or sodium lauryl sarcosinate and flavouring agents. A particularly preferred formulation includes, in addition to the phosphonomycin, saccharose, ammonium glycyrrhizinate, sodium lauryl sulphate, magnesium stearate, liquorice and mint in powder form.

As mentioned above, the tablets of the present invention dissolve slowly in the mouth, so that the ingestion of the drug is slow and the contact times with the various mucous membranes and tissues are substantially long, which results in better and more continuous absorption.

The present invention is now illustrated in more detail on the basis of three non-limiting examples.

### Example 1

1428 g of the calcium salt of phosphonomycin (corresponding to 1000 g of phosphonomycin in acid form) are formed into granules under moist conditions with 622 g of saccharose in 66% aqueous solution and the moist granules are dried at 40°C for one night.

To the dry granules obtained are added 14 g of ammonium glycyrrhizinate, 20 g of powdered liquorice, 234 g of powdered mint, 3.5 g of sodium lauryl sulphate and 11 g of magnesium stearate; the mixture obtained is homogenized and precompressed. The compressed granules are powdered and passed through a 12-mesh sieve after which a further 12 g of magnesium stearate are added, and then the product is compressed to form tablets of the desired form, about 1000 tablets being obtained each with a weight of 2.345 g and each containing 1 g of phosphonomycin, expressed in the form of the acid.

### Example 2

This is carried out substantially as described in Example 1, the difference being that 714 g of the calcium salt of phosphonomycin are used instead of 1428 g.

Thus about 1000 tablets, each having an individual weight of 1.630 g and each containing 0.5 g of phosphonomycin, expressed as the acid are obtained.

## Example 3

This is carried out in the manner described in Example 1, the doses of the individual ingredients being reduced to a quarter; thus about 1000 tablets each having an individual weight of 0.586 g and each containing 0.25 g of phosphonomycin, expressed in terms of the acid, are obtained.

The tablets prepared according to the present invention, left to dissolve slowly in the mouth, have dissolution times normally between about half an hour and an hour and a half, depending on the weight of the tablet.

### Claims

1. Pharmaceutical composition for the oral administration of the antibiotic 1(−)-cis-(1,2-epoxypropyl) phosphonic acid, characterised in that the said composition is in the form of a tablet to be sucked, containing an effective dose of 1(−)-cis-(1,2-epoxypropyl) phosphonic acid in the form of its calcium salt together with pharmaceutically acceptable excipients, containing the said calcium salt in quantities corresponding to 0.25 to 1.0 g of 1(−)-cis-(1,2-epoxypropyl) phosphonic acid, expressed in the form of the acid.

2. Pharmaceutical composition according to Claim 1, further including pharmaceutically acceptable sweeteners, flavouring agents, lubricants and/or dispersants.

### Patentansprüche

1. Pharmazeutische Zusammensetzung zur oralen Verabreichung des Antibiotikums 1(−)cis(1,2-Epoxypropyl)phosphonsäure, dadurch gekennzeichnet, daß sie in Form einer Lutschtablette vorliegt, die eine wirksame Dosis von 1(−)-cis(1,2-Epoxypropyl)phosphonsäure in Form ihres Calciumsalzes gemeinsam mit pharmazeutisch anwendbaren Bindmitteln enthält, wobei dieses Calciumsalz in Mengen vorliegt, die 0,25 bis 1,0 g 1(−)cis(1,2-Epoxypropyl)phosphonsäure, ausgedrückt als Säure, entsprechen.

2. Pharmazeutische Zusammensetzung nach Anspruch 1, die außerdem pharmazeutisch anwendbare Süßstoffe, Geschmacksmittel, Gleit- und/oder Dispergiermittel enthält.

### Revendications

1. Composition pharmaceutique pour l'administration orale de l'antibiotique acide 1(−)-cis-(1,2-époxypropyl)-phosphonique caractérisée en ce qu'elle se présente sous forme de comprimes à sucer contenant chacun une dose efficace d'acide 1(−)-cis-(1,2-époxy-propyl)-phosphonique sous forme de sel de calcium conjointement avec des excipients acceptables en pharmacie, ledit sel de calcium de acide 1(−)-cis-(1,2-époxypropyl)-phosphonique étant en quantité correspondant à 0,25—1,0 g d'acide 1(−)-cis-(1,2-époxypropyl)-phosphonique exprimée en acide.

2. Composition pharmaceutique selon la revendication 1, contenant aussi des édulcorants, des lubrifiants des dispersants et/ou des aromatisants acceptables en pharmacie.